# FASCICULE DE BREVET EUROPEEN

(11) **EP 0 601 911 B1**
(45) Date de publication et mention de la délivrance du brevet: **09.04.1997**
(21) Numéro de dépôt: 93402887.9
(22) Date de dépôt: 30.11.1993
(51) Int. Cl.: C07C 231/02, C07C 227/28, C07C 233/47, A61K 35/64, A61K 7/38, A01N 37/46, C11D 1/32

(54) **Acylaminoacides obtenus par l'acylation d'hydrolysats de protéines et compositions anti-microbiennes, antiparasitaires ou cosmétiques en contenant**
Durch Acylierung von Proteinhydrolipaten dargestellte Aminosäurederivate und antimikrobielle, antiparasitäre oder kosmetische Zusammensetzungen die diese enthalten
Acylamino acids obtained by acylation of protein hydrolipates and antimicrobial, antiparasitic or cosmetic compositions containing them

(30) Priorité: 09.12.1992 FR 9214840
(43) Date de publication de la demande: 15.06.1994
(73) Titulaire: LABORATOIRES PHYTOCOS, 27220 Grossoeuvre (FR)
(72) Inventeur: Morelle, Jean, F-75010 Paris (FR); Lauzanne, Eliane, F-75011 Paris (FR); Rothfuss, Jacqueline, F-67630 Lauterbourg (FR)

(56) Documents cités:
- FR-A- 2 289 179
- FR-A- 2 411 006
- FR-A- 2 422 400
- FR-A- 2 503 144
- CHEMICAL ABSTRACTS, vol. 102, no. 14, 8 Avril 1985, Columbus, Ohio, US; abstract no. 115009b, G. S. NADIGER ET AL. 'Investigation of amino acid composition in the crystalline region of silk fibroin' page 78 ; & J. APPL. POLYM. SCI. vol. 30, no. 1 , 1985 pages 221 - 225
- CHEMICAL ABSTRACTS, vol. 101, no. 8, 20 Aoüt 1984, Columbus, Ohio, US; abstract no. 59982d, 'Cosmetics containing oil-soluble silk peptides' page 295 ; & JP-A-59 084 808 (ICHIMARU FARUKOSU K. K.)
- DATABASE WPI Week 8621, Derwent Publications Ltd., London, GB; AN 86-133801 & JP-A-61 069 714 (SEIWA KASEI CO)

## Description

La présente invention concerne des nouveaux acylaminoacides qui sont des composés résultant de l'acylation, par un acide organique ou gras, de peptides ou d'acides aminés, obtenus par l'hydrolyse de certaines protéines.

L'invention concerne, plus particulièrement, les acylaminoacides obtenus en acylant des hydrolysats totaux de protéines contenant, essentiellement, de la fibroïne et de la séricine.

L'état de la technique, dans ce domaine, peut être illustré par les publications Chemical Abstract Volume 101, 1984, 59982d et Chemical Abstract Volume 102, 1985, 115009b, ainsi que par les brevets français No. 2 289 179, No. 2 411 006 et No. 2 422 400.

Les acylaminoacides et, plus spécialement, les lipoaminoacides obtenus par l'acylation, à l'aide d'une chaîne grasse, des acides aminés ou peptides courts, obtenus par hydrolyse des protéines animales, ont fait l'objet de nombreux brevets concernant aussi bien leur préparation que leurs différents emplois, principalement pour la cosmétique, la pharmacie et l'agriculture.

En cosmétique et en pharmacie, l'usage de ces composés est frappé d'une certaine suspicion, au même titre que les tissus animaux dont ils dérivent, du fait du risque de contamination par des agents pathogènes tels que les bactéries et les virus.

Aussi, récemment, pour éviter les risques de cette nature, s'est-on retourné vers les protéines végétales, plus particulièrement celles issues de graines d'oléagineux, caractérisées par leur richesse en acides aminés essentiels.

Ces acylaminoacides, préparés à partir de protéines animales ou végétales, présentent souvent l'inconvénient d'une odeur plus ou moins désagréable, limitant ainsi leur utilisation, principalement dans le domaine cosmétique.

La présente invention a pour objet de proposer de nouvelles structures acylaminoacides ne présentant pas de handicap sur le plan de l'odeur, qui sont obtenues à partir d'hydrolysats totaux de protéines contenant, essentiellement, de la fibroïne et de la séricine et, notamment, de préférence, des protéines sécrétées par le Bombyx Mori, qui correspond à la soie et qui contiennent des quantités significatives des structures polypeptidiques fibroïne et séricine. L'hydrolyse totale conduit à la libération des acides aminés qui, ensemble, représentent environ 90 % du total : la glycine, dont la teneur moyenne se situe entre 45 et 50 % 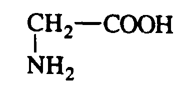 l'alanine, qui représente 28 à 30 % des chaînes polypeptidiques 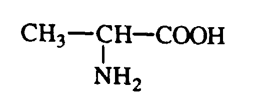 et la sérine, caractérisée par une fonction hydroxyle, dont la teneur se situe entre 10 et 15 %

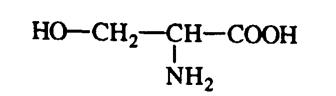

Quant aux autres acides aminés, leurs teneurs n'excèdent pas 5 % pour la tyrosine, 2 % pour la valine et 1 % pour les autres (cf. "The chemical structure and the crystalline structure of Bombyx Mori Silk fibroin" - LOTZ-COLONA CESARI - Biochimie 1979, (61), 205-214 et "HPLC fractionation of C₅ peptides of Bombyx Mori Silk Fibroin" - FREDDI, FARAGO et MAIFRENI, Sericologia 29 (3), 307-326).

Etant donné le volume relativement important occupé par les fibres, on effectue l'hydrolyse en deux temps. Pour la dégradation des fibres, dans un premier temps, trois parties des fibres sont plongées dans une solution contenant trois parties d'acide chlorhydrique à 33 % et une partie d'eau (parties en poids). Les fibres se dégradent facilement à une température de l'ordre de 80° C, ce qui réduit considérablement le volume des fibres mises en oeuvre.

Dans un deuxième temps, on effectue l'hydrolyse totale des fibres, ce qui nécessite environ 4 heures, pour obtenir une solution accusant une réaction biurétique négative.

L'hydrolysat est neutralisé par une solution de soude à 30 %, jusqu'à pH 5 environ, puis traité par un charbon actif pour la décoloration, qui sera d'autant plus aisée, qu'il y aura moins de cocons et de chrysalides, étant donné que, pour des raisons économiques, on aura recours à des déchets, constitués de fibres plus ou moins agglomérées, comportant des cocons ou des chrysalides.

On effectue le titrage de l'hydrolysat par formol titration, afin de pouvoir déterminer la quantité de chlorure ou d'anhydride, d'acides gras ou organiques nécessaires à l'acylation.

Dans le cas de chaînes grasses à partir de 5 atomes de carbone (on obtiendra des produits liposolubles), on effectue l'acylation en versant l'agent acylant dans l'hydrolysat, à des températures se situant entre 15 et 50° C, selon la nature de l'agent d'acylation, en maintenant le pH entre 10 et 11. Lorsque tout le réactif est introduit, on porte la température du milieu réactionnel à 70° C durant une heure, dans le cas de chlorure d'acides gras. On refroidit pour arriver à une température au dessous de 30° C, puis on traite par l'acide chlorhydrique, pour amener le milieu à pH 1/1,5. Il se forme une matte blanche, volumineuse, comportant en moyenne de 60 à 70 % d'eau. Cette matte sera lavée sur filtre, pour éliminer l'acide chlorhydrique, puis déshydratée sous vide, à une température inférieure à 50° C, pour éviter la coloration. Selon la nature de la chaîne grasse, on obtiendra des produits liposolubles, pouvant être réduits en poudre, ou pâteux.

Dans le cas de chaînes acyles comportant de 1 à 4 atomes de carbone, acétyle, propionyl, butyryl (on obtiendra des produits hydrosolubles), après avoir amené le pH du milieu vers 1 / 1,5, on élimine l'acide chlorhydrique issu de la réaction et l'eau, par distillation sous vide, l'acylaminoacide hydrosoluble étant extrait par un solvant comme l'éthanol, par exemple.

Les acylaminoacides réalisés selon l'invention sont des mélanges d'aminoacides acylés correspondant aux structures suivantes : 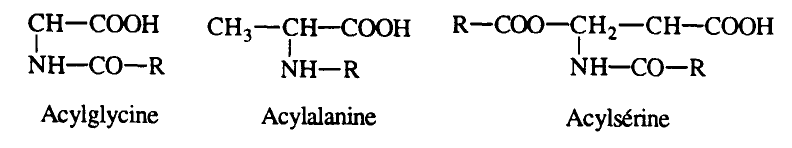 dans lesquelles R représente un radical d'acide gras de C₅ à C₃₀ ou d'un acide organique en C₁ à C₄. On obtient ainsi des acyles fibroïnoséricines, caractérisés par les trois acides aminés indiqués ci-dessus et par de faibles quantités des autres acides aminés classiques, qu'on révèle aisément en chromatographie sur couche mince et qui correspondent exactement aux spots obtenus sur l'hydrolysat de fibres de soie, caractérisés par les trois spots dominants de la glycine, de l'alanine et de la sérine.

### Exemples de préparations d'acylaminoacides selon l'invention :

### Exemple 1 - Hydrolyse de déchets de soie comportant des cocons :

Dans un réacteur contenant 300 ml d'acide chlorhydrique à 33 % et 100 ml d'eau, on introduit, au fur et à mesure de la dégradation des fibres, 300 g de déchets de soie. On porte la température vers 100° C et, lorsque le volume des fibres est suffisamment réduit - ce qui demande environ une heure - on porte à ébullition, durant environ 4 heures. Après refroidissement, l'hydrolysat est amené à pH 5 à l'aide d'une solution de soude, puis filtré pour séparer les éléments hydrocarbonés des cocons. Selon la teneur en cocons, on effectue la décoloration de l'hydrolysat par du charbon actif, soit à température ambiante, soit à 80° C. On détermine le titre de la solution par formol titration, on détermine le poids de l'agent acylant par rapport au contenu de fonctions NH₂ des acides aminés libérés par l'hydrolyse.

### Exemple 2 - Acylation par le chlorure de lauroyle :

A 200 ml d'hydrolysat de soie obtenu selon l'exemple 1, on ajoute, sous agitation, 400 ml d'eau et de la soude, pour porter le pH à 10,5. On fait couler goutte à goutte le chlorure de lauroyle, en maintenant le pH entre 10,5 et 11, la température se situant entre 30 et 40° C. Lorsque tout le chlorure a été introduit, on porte la température du milieu à 70° C, durant une heure.

Après refroidissement, on ajoute une solution d'acide chlorhydrique, pour porter le pH vers 1 / 1,5 ; l'acylaminoacide décante sous forme de matte blanchâtre, qui sera lavée à l'eau froide, puis déshydratée sous vide, à une température inférieure à 50° C, pour éviter des colorations. On obtient un produit fondant vers 89 / 90° C.

### Exemple 3 - Acylation par le chlorure de palmitoyl :

Comme pour l'exemple 2, à 200 ml d'hydrolysat de soie obtenu selon l'exemple 1, on ajoute, dans les mêmes conditions de pH, 96 g de chlorure de palmitoyl. Après acylation et chauffage à 70° C durant environ une heure, on refroidit le milieu et on libère le dérivé par l'acide chlorhydrique. La matte est lavée à froid, puis séchée sous vide. On obtient une poudre fondant à 98 / 99° C.

### Exemple 4 - Acylation par l'anhydride acétique :

200 ml d'hydrolysat de soie obtenu selon l'exemple 1 sont portés à pH 10,5 et à une température ne dépassant pas 20° C, le milieu réactionnel étant maintenu à cette température par de la glace. Sous agitation, on introduit, goutte à goutte, 98 g d'anhydride acétique, en maintenant le pH entre 10,5 et 11. Lorsque tout l'anhydride a été ajouté, l'agitation est maintenue durant 30 minutes, puis on acidifie le mélange par HCl à 33 %, jusqu'à obtenir un pH de 1 à 1,5. L'eau et l'acide chlorhydrique excédentaire sont éliminés sous pression réduite. On obtient finalement un mélange de chlorure de sodium et d'acétyl-fibroïne-séricine qui, repris par un solvant comme l'alcool, permet d'éliminer, par filtration, le chlorure de sodium. L'acylaminoacide cristallise dans l'alcool, sous forme de petits cristaux, formant une masse cireuse fondant à 59 / 60° C.

Le tableau ci-après indique les différentes caractéristiques analytiques des acylaminoacides réalisés selon l'invention :

| Acyles | Acétyle | Octanoyl | Undécylénoyl | Lauroyl | Palmitoyl | Oléoyl | Linoléoyl |
|---|---|---|---|---|---|---|---|
| Nitrogène | 7,5 / 8 | 5,8 / 6,5 | 4 / 4,5 | 3,8 / 4,4 | 3,5 / 4 | 2,5 / 2,8 | 1,8 / 2 |
| Indice d'acide | 370 | 274/ 285 | 230 / 240 | 220 / 240 | 275 / 290 | 140 / 160 | 115 / 130 |

L'invention concerne également des compositions diverses préparées avec les acylaminoacides de l'invention, lesdites compositions pouvant être utilisées dans les domaines cosmétique, hygiénique, thérapeutique et en agriculture.

Les nouveaux acylaminoacides peuvent être utilisés tels quels, i.e. sous leur forme acylée, ou sous la forme de leurs sels respectifs avec des alcalino-métaux ou des bases organiques, tels que les différents acides aminés ou les alcools aminés, ou des bases biologiques telles que la lysine, la choline ou l'ammoniaque ; les métaux alcalino-terreux peuvent également être utilisés, tout comme les métaux généralement appelés oligo-éléments, tels le fer, le manganèse, le cobalt et le cuivre.

Le choix de l'agent utilisé pour la formation de sels dépendra, naturellement, de l'usage auquel le composé est destiné.

Par exemple, pour la réalisation de compositions détergentes, on préfèrera la salification par une base minérale, organique ou biologique, de l'acylaminoacide, dont la fraction acyle correspond à la chaîne laurique, comme indiqué dans l'exemple 2. On obtient ainsi un produit principalement destiné à l'hygiène corporelle qui, à la teneur de 20 %, est caractérisé par un pouvoir moussant jamais atteint avec les autres acides aminés issus d'hydrolysats animaux ou végétaux, par une douceur exceptionnelle très caractéristique et par un puissant pouvoir nettoyant, ces propriétés étant dues aux faibles poids moléculaires des acides aminés dont, principalement, la glycine. Comme bases minérales, on pourra utiliser la soude ou la potasse, comme bases organiques, la monoéthanolamine et, comme bases biologiques, la lysine, la choline ou l'ammoniaque.

On réalisera avantageusement des compositions antisudorales, capables d'inhiber l'hyperhidrose, en ayant recours aux sels d'aluminium mono et dibasique, avec des chaînes acyles en C₈ et C₁₁.

Les acylaminoacides réalisés selon l'invention permettent d'obtenir des substances particulièrement intéressantes dans le domaine cosmétique, du fait de comporter de la sérine, un des principaux constituants de la soie, qui, sous la forme acylée, présente l'avantage d'augmenter la distribution de l'eau dans des régions déshydratées ou apolaires (A. KAPLAN : "Hydrogene bonding properties of N-Palmitoyl-Sérine" - Journal of colloid and interface Science, 1967 (25), 63-70) et d'être antitoxiques (MAC FARLANE : Adv Lipid Res., 1964 (2), 96-125).

On réalisera, à partir de la présente invention, des compositions antimicrobiennes, antifongiques et antivirales (agent acylant en C₈ ou C₁₁, forme acylée), dans ce dernier cas, par exemple, actives sur l'herpès.

Dans le domaine de l'hygiène tel que la protection contre les poux (antipédiculosiques *capitis et corporis*), on aura recours à des chaînes acyles comportant de 3 à 11 atomes de carbone, avec des acylaminoacides acylés.

En agriculture, on utilisera avantageusement les acylaminoacides en C₈ et C₂₂ décrits, salifiés par des oligo-éléments dont, notamment, le cuivre, comme agents de protection des végétaux, ou comme agents de stimulation de développement.

En pharmacie, on réalisera des compositions anti-inflammatoires (agent acylant C₈ à C₂₂, forme acylée), dans le traitement des eczémas et de l'intertrigo, par exemple, dans le traitement de toutes les inflammations microbiennes, fongiques ou virales de la peau.

A titre d'exemples non limitatifs de compositions diverses réalisées selon l'invention, citons :

### 1 - Composition cosmétique (crème) :

| | |
|---|---|
| Acide stéarique | 8 |
| Alcool cétylique | 2 |
| Stéarate de polyoxyéthylèneglycol | 5 |
| Palmitoyl fibroïne séricine | 4 |
| Glycérine | 10 |
| Eau | qs100 |

### 2 - Composition nettoyante pour l'hygiène corporelle :

| | |
|---|---|
| Lauroyl fibroïne séricine sodique à 20 % d'extrait sec | 30 |
| Octanoyl fibroïne séricine | 0,5 |
| NaCl pour augmentation de la viscosité | 5 |
| Eau déminéralisée | qs100 |

### 3 - Savon de toilette :

| | |
|---|---|
| Copeaux de savon de coprah | 95 |
| Lauroyl fibroïne séricine | 5 |

### 4 - Composition antitranspirante :

| | |
|---|---|
| Undecylenoyl-fibroïne séricine salifié par l'aluminium sous forme dibasique | 5 |
| Palmitostéarate de glycérol | 5 |
| Alcool cétylique polyoxyéthyléné | 8 |
| Propylèneglycol | 10 |
| Eau | qs100 |

### 5 - Composition antipédiculose :

| | |
|---|---|
| Octanoyl-fibroïne-séricine | 3 |
| Alcool à 30° | 97 |

### 6 - Composition antiseptique antiacnéique :

| | |
|---|---|
| Octanoyl-fibroïne-séricine | 4 |
| Alcool gras polyoxyéthyléné | 5 |
| Eau | 91 |

### 7 - Composition pour la protection des végétaux :

| | |
|---|---|
| Octanoyl-fibroïne-séricine salifié par du cuivre | 5 |
| Solution d'ammoniaque à 30 % | 5 |
| Eau | qs100 |

## Revendications

1. Acylaminoacides résultant de l'acylation, par un radical acyle comportant de 1 à 30 atomes de carbone, d'un mélange d'acides aminés obtenus par hydrolyse totale d'une protéine caractérisée en ce qu'elle contient des quantités significatives de fibroïne et de séricine.

2. Acylaminoacides selon la revendication 1, caractérisés en ce que la protéine est le Bombyx Mori.

3. Acylaminoacides selon l'une des revendications 1 et 2, caractérisés en ce que la protéine provient de déchets de soie, sous forme de fibres comportant ou non des cocons ou des chrysalides.

4. Acylaminoacides selon l'une des revendications 1 à 3, caractérisés en ce que le mélange d'acides aminés à acyler contient essentiellement de la glycine, de l'alanine et de la sérine, ces trois acides aminés représentant 90 % de l'ensemble des acides aminés, avec des proportions comprises entre 45 et 60 % pour la glycine, entre 20 et 30 % pour l'alanine et entre 10 et 15 % pour la sérine.

5. Acylaminoacides selon l'une des revendications 1 à 4, caractérisés en ce qu'ils sont salifiés par les bases minérales soude ou potasse, ou par les bases organiques ammoniaque, éthanolamines ou les bases biologiques lysine ou choline.

6. Acylaminoacides selon la revendication 5, caractérisés en ce que l'agent acylant est la chaîne laurique en C₁₂, pour la préparation de compositions détergentes à usage corporel.

7. Acylaminoacides selon l'une des revendications 1 à 4, caractérisés en ce qu'ils sont salifiés par des métaux dits oligo-éléments, par des alcalino terreux ou, encore, par l'aluminium.

8. Compositions cosmétiques caractérisées en ce qu'elles contiennent des acylaminoacides selon les revendications 1 à 7.

9. Compositions antisudorales, contenant des acylaminoacides selon la revendication 7, caractérisées en ce que le métal est l'aluminium, sous la forme monobasique ou dibasique.

10. Compositions anti-microbiennes et antifongiques, contenant des acylaminoacides selon les revendications 1 à 4, caractérisées en ce que la chaîne acyle correspond à un radical octanoyle ou undécylénoyle.

11. Compositions hygiéniques présentant des propriétés antiparasitaires et contenant des acylaminoacides selon les revendications 1 à 4, caractérisées en ce que la chaîne acyle correspond à un radical octanoyle ou undécylénoyle.

12. Compositions médicamenteuses destinées aux traitements des inflammations cutanées, contenant des acylaminoacides selon les revendications 1 à 4, caractérisées en ce que l'agent acylant comprend de 8 à 22 atomes de carbone.

13. Compositions pour l'agriculture, contenant des acylaminoacides salifiés par des oligo-éléments selon la revendication 7, destinées à la stimulation du développement des plantes et à leur protection contre certains parasites, caractérisées en ce que l'agent acylant comprend de 8 à 22 atomes de carbone.

## Patentansprüche

1. Acylaminosäuren, die sich aus der Acylierung durch einen Acylradikal mit 1 bis 30 Kohlenstoffatomen aus einem durch Totalhydrolyse eines Proteins, das sich durch einen beträchtlichen Fibroin- und Sericingehalt auszeichnet, gewonnenen Aminosäurengemisch ergeben.

2. Acylaminosäuren gemäß der Forderung 1, gekennzeichnet dadurch, daß es sich bei dem Protein um Bombyx Mori *(Maulbeer-Seidenspinner)* handelt.

3. Acylaminosäuren gemäß einer der Forderungen 1 und 2, gekennzeichnet dadurch, daß das Protein aus Abfallseide in Form von Fasern stammt, die auch die Kokons oder Chrysalide enthalten können.

4. Acylaminosäuren gemäß einer der Forderungen 1 bis 3, gekennzeichnet dadurch, daß das zu acylierende Aminosäurengemisch vorwiegend Glycin, Alanin und Serin enthält, Diese drei Aminosäuren müssen 90 Gew.-% der gesamten Aminosäuren enthalten, wobei der Anteil für Glycin zwischen 45 und 60 Gew.-%, für Alanin zwischen 20 und 30 Gew.-% und für Serin zwischen 10 und 15 Gew.-% liegen muß.

5. Acylaminosäuren gemäß einer der Forderungen 1 bis 4, gekennzeichnet dadurch, daß sie durch Natriumcarbonat- oder Kali-Mineralbasen, oder organische Ammoniakbasen, Ethanolamine oder Lysin- bzw. Cholin-Biobasen, in Salze umgewandelt wurden.

6. Acylaminosäuren gemäß der Forderung 5, gekennzeichnet dadurch, daß es sich bei dem acylierenden Agens um eine C₁₂-Laurin(*säure*)kette handelt, für die Herstellung von Seifepräparaten für die Körperpflege.

7. Acylaminosäuren gemäß einer der Forderungen 1 bis 4, gekennzeichnet dadurch, daß sie durch mit Spurenelemente bezeichnete Metalle, Erdalkalimetalle oder auch Aluminium in Salze umgewandelt wurden.

8. Kosmetische Präparate, gekennzeichnet dadurch, daß sie gemäß den Forderungen 1 bis 7 Acylaminsäuren enthalten.

9. Gemäß der Forderung 7 Acylaminosäuren enthaltende schweißhemmende Präparate, gekennzeichnet dadurch, daß es sich bei dem Metall um Aluminium in mono- oder dibasischer Form handelt.

10. Gemäß den Forderungen 1 bis 4 Acylaminosäuren enthaltende Mikrobizid- und Fungizidpräparate, gekennzeichnet dadurch, daß die Acyl-Kette dem Octanoyl- oder Undecylenoyl-Radikal entspricht.

11. Gemäß den Forderungen 1 bis 4 Acylaminosäuren enthaltende Körperpflegepräparate mit parasitiziden Eigenschaften, gekennzeichnet dadurch, daß die Acyl-Kette dem Octanoyl- oder Undecylenoyl-Radikal entspricht.

12. Gemäß den Forderungen 1 bis 4 Acylaminosäuren enthaltende Arzneimittelpräparate zur Behandlung von Hautentzündungen, gekennzeichnet dadurch, daß das acylierende Agens 8 bis 22 Kohlenstoffatome enthält.

13. Gemäß der Forderung 7 durch Spurenelemente in Salze umgewandelte Acylaminosäuren enthaltende landwirtschaftliche Zubereitungen für die Wachstumförderung von Pflanzen und deren Schutz gegen Parasiten, gekennzeichnet dadurch, daß das acylisierende Agens 8 bis 22 Kohlenstoffatome enthält.

## Claims

1. Acylamino acids resulting from the acylation, by an acyl radical comprising of from 1 to 30 carbon atoms, of a mixture of amino acids obtained by total hydrolysis of a protein characterised by the fact that it comprises significant quantities of fibroin and sericin.

2. Acylamino acids according to claim 1, characterised by the fact that the protein is Bombyx Mori.

3. Acylamino acids according to one of claims 1 and 2, wherein the protein is issued from silk waste, under the form of fibres comprising or not cocoons or chrysalides.

4. Acylamino acids according to one of claims 1 to 3, characterised by the fact that the mixture of amino acids which have to be acylated essentially contains glycine, alanine and serine, these three amino acids representing 90 % of the total of amino acids, in proportions comprised between 45 and 60 % for glycine, between 20 and 30 % for alanine and between 10 and 15 % for serine.

5. Acylamino acids according to one of claims 1 to 4, characterised by the fact that they are salified by the mineral bases soda or potash, or by organic bases ammonia, ethanol amines or by biological bases lysine or choline.

6. Acylamino acids according to claim 5, characterised by the fact that the acylating agent is the lauric chain in C₁₂, for the preparation of detergent compositions for bodily use.

7. Acylamino acids according to one of claims 1 to 4, characterised by the fact that they are salified by metals called oligo-elements, by alkaline-earth metals or, else, by aluminium.

8. Cosmetic compositions, characterised by the fact that they comprise acylamino acids according to claims 1 to 7.

9. Anti-sudoral compositions containing acylamino acids according to claim 7, wherein the metal is aluminium, under the monobasic or dibasic form.

10. Anti-microbial and anti-fungic compositions containing acylamino acids according to claims 1 to 4, wherein the acyl chain corresponds to an octanoyl or undecylnoyl radical.

11. Hygienic compositions presenting anti-parasitic properties and containing acylamino acids according to claims 1 to 4, wherein the acyl chain corresponds to an octanoyl or undecylnoyl radical.

12. Medicinal compositions for the treatment of cutaneous inflammations, containing acylamino acids according to claims 1 to 4, characterised by the fact that the acylating agent comprises of from 8 to 22 carbon atoms.

13. Compositions for agriculture, containing acylamino acids salified by oligo-elements according to claim 7, used for the stimulation of the development of plants and for their protection against some parasites, characterised by the fact that the acylating agent comprises of from 8 to 22 carbon atoms.
